(19) Europäisches Patentamt
European Patent Office
Office européen des brevets

(11) EP 0 711 752 A2

(12) **EUROPÄISCHE PATENTANMELDUNG**

(43) Veröffentlichungstag:
**15.05.1996 Patentblatt 1996/20**

(51) Int. Cl.$^6$: **C07C 255/37**, C07C 253/34

(21) Anmeldenummer: **95116377.3**

(22) Anmeldetag: **18.10.1995**

(84) Benannte Vertragsstaaten:
**BE CH DE ES FR GB IE IT LI NL**

(30) Priorität: **08.11.1994 DE 4439836**

(71) Anmelder: **HOECHST AKTIENGESELLSCHAFT**
**D-65929 Frankfurt am Main (DE)**

(72) Erfinder:
- **Beller, Matthias, Dr.**
  **D-65510 Idstein (DE)**
- **Pfirmann, Ralf, Dr.**
  **D-64347 Griesheim (DE)**

(54) **Cis-4-(2,2,3,3-Tetrafluorpropoxy)-zimtsäurenitril und trans-4-(2,2,3,3-Tetrafluorpropoxy)-zimtsäurenitril und ein Verfahren zu ihrer Herstellung**

(57) Die vorliegende Erfindung betrifft die Verbindungen cis-4-(2,2,3,3-Tetrafluorpropoxy)-zimtsäurenitril und trans-4-(2,2,3,3-Tetrafluorpropoxy)-zimtsäurenitril und ein Verfahren zu ihrer Herstellung. Das Verfahren betrifft die Herstellung von cis-4-(2,2,3,3-Tetrafluorpropoxy)-zimtsäurenitril und trans-4-(2,2,3,3-Tetrafluorpropoxy)-zimtsäurenitril oder wahlweise einer dieser beiden Verbindungen, indem man ein cis- und trans-4-(2,2,3,3-Tetrafluorpropoxy)-zimtsäurenitril enthaltendes Gemisch unter reduziertem Druck fraktioniert destilliert, eine mindestens 80 Gew.-% cis-4-(2,2,3,3-Tetrafluorpropoxy)-zimtsäurenitril und eine mindestens 80 Gew.-% trans-4-(2,2,3,3-Tetrafluorpropoxy)-zimtsäurenitril enthaltende Hauptfraktion abtrennt, diese Hauptfraktionen mittels fraktionierter Kristallisation oder Schmelzkristallisation weiter reinigt oder eine dieser Hauptfraktionen durch Isomerisierung auf das dem jeweiligen thermodynamischen Gleichgewicht entsprechende cis-/trans-Isomerenverhältnis einstellt und in das Verfahren zurückführt und die übrigen Fraktionen direkt oder nach Isomerisierung in das Verfahren zurückführt.

EP 0 711 752 A2

## Beschreibung

Die vorliegende Erfindung betrifft die Verbindungen cis-4-(2,2,3,3-Tetrafluorpropoxy)-zimtsäurenitril und trans-4-(2,2,3,3-Tetrafluorpropoxy)-zimtsäurenitril sowie ein Verfahren zu ihrer Herstellung.

Nach Ann. N.Y. Acad. Sci (1988), 544, Seiten 86 bis 100 (Chem. Abstr. 111, 208650) läßt sich 4-Tetrafluorpropoxyzimtsäurenitril folgendermaßen herstellen. 4-Fluorbenzonitril wird mit 2,2,3,3-Tetrafluorpropanol zu 4-(2,2,3,3-Tetrafluorpropoxy)-benzonitril umgesetzt, das anschließend mittels komplexer Hydride zu 4-(2,2,3,3-Tetrafluorpropoxy)-benzaldehyd reduziert wird. Der 4-(2,2,3,3-Tetrafluorpropoxy)-benzaldehyd führt durch Reaktion mit dem Anion des Cyanomethylphosphonsäurediethylesters im Sinne einer Wittig-Horner Reaktion zum 4-Tetrafluorpropoxyzimtsäurenitril.

Dabei fällt jedoch das 4-Tetrafluorpropoxyzimtsäurenitril stets als Gemisch der geometrischen Isomeren, also als cis-/trans-Isomerengemisch an. Dieses cis-/trans-Isomerengemisch kann als Ausgangsmaterial für die technische Herstellung von Antimykotika verwendet werden. Hierbei müssen allerdings sämtliche Nachteile, die mit dem Gebrauch eines cis-/trans-Isomerengemisches zwangsweise verbunden sind, in Kauf genommen werden. Als Nachteile gegenüber einer isomererenreinen Verbindung seien beispielsweise die durch die Herstellung und Aufarbeitung bedingten wechselnden Gehalte an den jeweiligen geometrischen Isomeren sowie die unterschiedliche Wirksamkeit der geometrischen Isomeren in dem jeweiligen pharmazeutisch wirksamen Endprodukt erwähnt.

Isomerenreine Derivate der 4-(2,2,3,3-Tetrafluorpropoxy)-zimtsäure, insbesondere das (E)-4-(2,2,3,3-Tetrafluorpropoxy)-zimtsäureamid und ein aus ihm herstellbares Triazolderivat, werden bei der Herstellung von Fungiziden eingesetzt (EP 0 472 392). Der dabei beschrittene Syntheseweg ist allerdings mit einem sehr hohen Aufwand verbunden.

In einem ersten Schritt wird p-Chlorbenzonitril mit 2,2,3,3-Tetrafluorpropanol in Gegenwart von Basen, beispielsweise Natriumhydrid unter Austausch des p-Chloratoms zu 4-(2,2,3,3-Tetrafluorpropoxy)-benzonitril umgesetzt. Der zweite Schritt besteht darin, das 4-(2,2,3,3-Tetrafluorpropoxy)-benzonitril mittels Metallhydriden, beispielsweise Diisobutylaluminiumhydrid, zu reduzieren, wobei der 4-(2,2,3,3-Tetrafluorpropoxy)-benzaldehyd entsteht. Der 4-(2,2,3,3-Tetrafluorpropoxy)-benzaldehyd wird in einem dritten Reaktionsschritt mit Ethoxycarbonylmethanphosphonsäuremethyldiethylester stereoselektiv zu (E)-4-(2,2,3,3-Tetrafluorpropoxy)-zimtsäureethylester umgesetzt.

In einer nachfolgenden weiteren Reaktionsstufe wird der (E)-4-(2,2,3,3-Tetrafluorpropoxy)-zimtsäureethylester mit wäßriger Natronlauge verseift und die (E)-4-(2,2,3,3-Tetrafluorpropoxy)-zimtsäure durch Ansäuern und Extrahieren gewonnen.
Aus der (E)-4-(2,2,3,3-Tetrafluorpropoxy)-zimtsäure erhält man durch Reaktion mit Thionylchlorid das entsprechende (E)-4-(2,2,3,3-Tetrafluorpropoxy)-zimtsäurechlorid (fünfter Schritt), das in einem abschließenden sechsten Schritt durch Umsetzung mit Ammoniak das (E)-4-(2,2,3,3,-Tetrafluorpropoxy)-zimtsäureamid ergibt. Aus dem (E)-4-(2,2,3,3-Tetrafluorpropoxy)-zimtsäureamid wird anschließend unter Ringschluß an der Säureamidgruppe ein Triazolderivat, nämlich 3-[(E)-4-(2,2,3,3-Tetrafluorpropoxy)-styryl]-1H-1,2,4-triazol, gebildet. Das Triazol dient als wichtiger Baustein für die Herstellung von Fungiziden. Der vorstehend geschilderte Syntheseweg ist in der EP 0 472 392 auf Seite 10, Zeilen 42 bis Seite 11, Zeile 6 sowie in den Beispielen 1 bis 6 auf Seite 14 bis 16 näher beschrieben.

Die vorangegangenen Ausführungen belegen, daß generell ein lebhaftes Interesse besteht, 4-(2,2,3,3-Tetrafluorpropoxy)-zimtsäurederivate in isomerenreiner Form herzustellen und weiter zu verarbeiten. Hierbei wird auch, wie in der EP 0 472 392 beschrieben, ein sehr komplizierter und technisch aufwendiger Syntheseweg in Kauf genommen.

Vor diesem Hintergrund stellt es eine lohnende Aufgabe dar, weitere 4-(2,2,3,3-Tetrafluorpropoxy)-zimtsäurederivate in isomerenreiner oder weitgehend isomerenreiner Form bereitzustellen.

Gelöst wird diese Aufgabe durch die Verbindungen cis-4-(2,2,3,3-Tetrafluorpropoxy)-zimtsäurenitril und trans-4-(2,2,3,3-Tetrafluorpropoxy)zimtsäurenitril.

Zum leichteren Verständnis sind nachfolgend beide Verbindungen noch mit ihren Formeln wiedergegeben:
cis-4-(2,2,3,3-Tetrafluorpropoxy)-zimtsäurenitril (A)

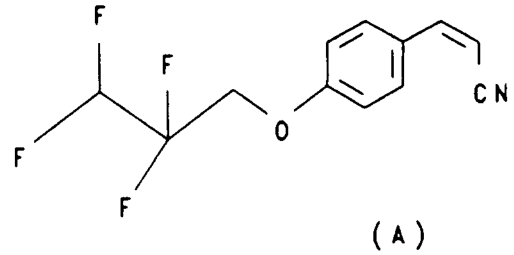

(A)

und trans-4-(2,2,3,3-Tetrafluorpropoxy)-zimtsäurenitril (B)

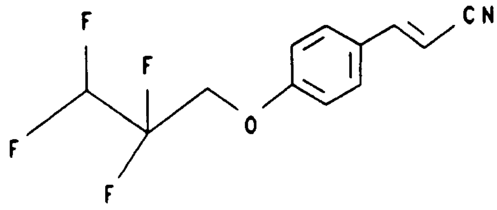

(B)

Aus diesem cis- und trans-Nitril läßt sich, beispielsweise durch Wasseranlagerung, das entsprechende cis- und trans-4-(2,2,3,3-Tetrafluorpropoxy)-zimtsäureamid herstellen.

Weiterhin besteht eine interessante Aufgabe darin, die Verbindung cis-4-(2,2,3,3-Tetrafluorpropoxy)-zimtsäurenitril und trans-4-(2,2,3,3-Tetrafluorpropoxy)-zimtsäurenitril auf einfache Art und ausgehend von einem vergleichsweise leicht zugänglichen Ausgangsstoff in isomerenreiner oder weitgehend isomerenreiner Form zugänglich zu machen.

Diese Aufgabe wird gelöst durch ein Verfahren zur Herstellung von cis-4-(2,2,3,3-Tetrafluorpropoxy)-zimtsäurenitril und trans-4-(2,2,3,3-Tetrafluorpropoxy)-zimtsäurenitril oder wahlweise einer dieser beiden Verbindungen. Es ist dadurch gekennzeichnet, daß man ein cis- und trans-4-(2,2,3,3-Tetrafluorpropoxy)-zimtsäurenitril enthaltendes Gemisch unter reduziertem Druck fraktioniert destilliert, eine mindestens 80 Gew.-% cis-4-(2,2,3,3-Tetrafluorpropoxy)-zimtsäurenitril und eine mindestens 80 Gew.-% trans-4-(2,2,3,3-Tetrafluorpropoxy)-zimtsäurenitril enthaltende Hauptfraktion abtrennt, diese Hauptfraktionen mittels fraktionierter Kristallisation oder Schmelzkristallisation weiter reinigt oder eine dieser Hauptfraktionen durch Isomerisierung auf das dem jeweiligen thermodynamischen Gleichgewicht entsprechende cis-/trans-Isomerenverhältnis einstellt und in das Verfahren zurückführt und die übrigen Fraktionen direkt oder nach Isomerisierung in das Verfahren zurückführt.

Ein wichtiger Vorteil des erfindungsgemäßen Verfahrens besteht darin, daß man nicht auf eine sehr aufwendige, stereoselektive Synthese, bei der von vorneherein festgelegt wird, ob das cis- oder das trans-4-(2,2,3,3-Tetrafluoropropoxy)-zimtsäurenitril hergestellt wird, angewiesen ist. Vielmehr kann man ein beliebiges cis- und trans-4-(2,2,3,3-Tetrafluorpropoxy)-zimtsäurenitril enthaltendes Gemisch als Ausgangsmaterial verwenden.

Derartige Gemische sind nach einem vergleichsweise recht einfachen Verfahren, das Gegenstand einer nicht vorveröffentlichten Anmeldung (DE 44 08 083) ist, in guter Ausbeute zugänglich.

Der Vollständigkeit halber sei auf dieses Verfahren an dieser Stelle etwas näher eingegangen.

Die DE 44 08 083 betrifft ein Verfahren zur Herstellung von 4-Fluoralkoxyzimtsäurenitrilen der Formel (I)

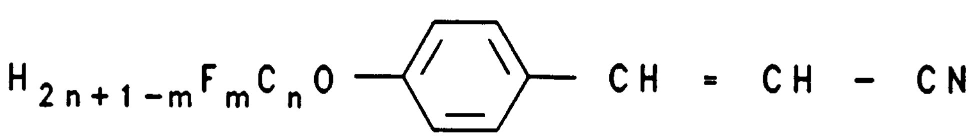

(I)

worin n = 1 bis 8 und m = 1 bis 17 sind, wobei $m \leq 2n + 1$ ist, indem man 4-Fluorbenzaldehyd in Gegenwart einer Base und gegebenenfalls eines Lösungsmittels mit einem Fluoralkanol der Formel (II),

$$H_{2n+1-m}F_mC_nO\text{-}H \qquad (II)$$

worin m und n die oben angegebene Bedeutung besitzen, umsetzt und den erhaltenen 4-Fluoralkoxybenzaldehyd mit Cyanessigsäure oder einem Cyanessigsäurealkylester in Gegenwart einer Base und gegebenenfalls eines Lösungsmittels umsetzt.

Das Verfahren läßt sich folgendermaßen durchführen:

4-Fluorbenzaldehyd wird mit dem Fluoralkanol in Gegenwart von 0,5 bis 3, insbesondere 0,6 bis 1,25 Äquivalenten Base bei Temperaturen zwischen 10°C und 180°C, insbesondere 60 bis 155°C zum entsprechenden 4-Fluoralkoxybenzaldehyd umgesetzt. Als Base eignen sich z.B. Alkalicarbonate, insbesondere Kaliumcarbonat, Natriumcarbonat oder Gemische aus Kaliumcarbonat und Natriumcarbonat. Die Umsetzung kann sowohl in Gegenwart von dipolar aprotischen

Lösungsmitteln wie N,N-Dimethylacetamid, Sulfolan und N,N-Dimethylformamid als auch lösungsmittelfrei durchgeführt werden. Im letzteren Falle wird vorteilhaft in überschüssigem Fluoralkanol gearbeitet, das letztlich als Lösungsmittel fungiert. Die Einsatzmenge an Fluoralkanol ist abhängig von der eingesetzten Menge an Base so zu wählen, daß das Reaktionsgemisch rührbar bleibt und beträgt bezogen auf eingesetzten 4-Fluorbenzaldehyd vorteilhaft 0,8 bis 1,5 Äquivalente.

Für die Aufarbeitung des anfallenden Reaktionsgemisches bieten sich zwei Wege an:

1) Das Reaktionsgemisch wird durch Filtration von den vorhandenen Salzen (überschüssige Base und gebildetes Basenfluorid) abgetrennt. Anschließend wird das Produkt aus dem Filtrat destilliert.

2) Alternativ dazu ist es möglich, durch Zusatz von Wasser das gebildete Basenfluorid und im Überschuß vorhandenes Salz aufzulösen, die gebildeten Phasen zu trennen und das in der organischen Phase befindliche Produkt entweder direkt in die Folgeumsetzung einzubringen oder im Bedarfsfall destillativ zu reinigen. In der wäßrigen Phase befindliches Produkt kann mit Lösungsmitteln wie Toluol, Chlorbenzol, Dichlorbenzol, Methylenchlorid oder tert.-Butylmethylether extrahiert werden.

Der erhaltene 4-Fluoralkoxybenzaldehyd wird anschließend mit 0,3 bis 4 Äquivalenten, vorzugsweise mit 0,6 bis 2,0 Äquivalenten, bevorzugt mit 0,8 bis 1,2 Äquivalenten Cyanessigsäure oder Cyanessigsäurealkylestern bei Temperaturen von 50 bis 250°C, vorzugsweise 80 bis 180°C in Gegenwart einer Base oder eines basischen Katalysators zum 4-Fluoralkoxyzimtsäurenitril umgesetzt.

Als Cyanessigsäurealkylester haben sich Cyanessigsäuremethylester, Cyanessigsäureethylester und Cyanessigsäurepropylester als günstig erwiesen. Als Base oder basischer Katalysator können aromatische und aliphatische Amine, Alkali- und Erdalkalicarbonate oder basische Oxide und Hydroxide wie NaOH, KOH oder $Al_2O_3$ eingesetzt werden.

Als Base und gegebenenfalls auch als Lösungsmittel haben sich in vielen Fällen Amine wie Pyridin, Piperidin, Morpholin, Tri-butylamin, Triethylamin, Tripropylamin, Benzylamin, Anilin, Dialkylanilin bewährt.

Die Umsetzung mit Cyanessigsäurederivaten kann in Abwesenheit von Lösungsmitteln durchgeführt werden. Es kann jedoch auch vorteilhaft sein, in Anwesenheit von Lösungsmitteln zu arbeiten. Als Lösungsmittel können beispielsweise Aromaten wie Benzol, Toluol, Xylole, dipolar aprotische Lösungsmittel wie Dimethylformamid, Dimethylacetamid, Dimethylsulfoxid, Sulfolan, Alkohole wie Ethanol, Propanol, Butanol, Glyme oder Ether wie Diglyme dienen. Die Menge an Lösungsmittel beträgt, bezogen auf den eingesetzten 4-Fluoralkoxybenzaldehyd, 5 bis 90 Gew.-%.

Zur Herstellung von 4-(2,2,3,3-Tetrafluorpropoxyl)-zimtsäurenitril setzt man in das vorstehend geschilderte Verfahren 4-Fluorbenzaldehyd mit 2,2,3,3-Tetrafluorpropanol als Fluoralkanol um und verfährt ansonsten wie zuvor erläutert.

Man erhält auf diese Weise ein cis-4-(2,2,3,3-Tetrafluorpropoxy)-zimtsäurenitril und trans-4-(2,2,3,3-Tetrafluorpropoxy)-zimtsäurenitril enthaltendes Gemisch, das in das erfindungsgemäße Verfahren als Ausgangsmaterial eingesetzt werden kann.

Neben der Verwendung eines cis-/trans-Isomerengemisches besitzt das erfindungsgemäße Verfahren noch einen weiteren erheblichen Vorteil. Es gestattet nämlich, je nach Wunsch und Bedarf nicht nur das cis-4-(2,2,3,3-Tetrafluorpropoxy)-zimtsäurenitril oder trans-4-(2,2,3,3-Tetrafluorpropoxy)-zimtsäurenitril, sondern auch ausschließlich oder überwiegend eines der beiden Isomeren zu Lasten des jeweils nicht benötigten Isomeren herzustellen. Wird beispielsweise das cis-Isomere benötigt, so ermöglicht das erfindungsgemäße Verfahren eine Umwandlung des trans-4-(2,2,3,3-Tetrafluorpropoxy)-zimtsäurenitrils in das entsprechende cis-Isomere und dessen anschließende Abtrennung als Reinprodukt. Wird andererseits das trans-Isomere gewünscht, so ermöglicht das erfindungsgemäße Verfahren eine Umwandlung des cis-4-(2,2,3,3-Tetrafluorpropoxy)-zimtsäurenitrils in das trans-Isomere und dessen abschließende Abtrennung als Reinprodukt.

Dadurch ist eine sehr flexible Anpassung an den jeweiligen Bedarf des einen oder anderen Isomeren möglich, ohne daß das jeweils nicht benötigte Isomere als unerwünschtes Nebenprodukt anfällt.

Das erfindungsgemäße Verfahren läßt sich auf Gemische, die cis- und trans-4-(2,2,3,3-Tetrafluorpropoxy)-zimtsäurenitril in beliebigen Verhältnis enthalten, anwenden. Je nach Zusammensetzung des als Einsatzstoff verwendeten Gemisches erhält man das cis- und das trans-4-(2,2,3,3-Tetrafluorpropoxy)-zimtsäurenitril in einer Reinheit von wenigstens 95, insbesondere 97,5 %. Es lassen sich jedoch auch Reinheiten von 99 % und darüber erzielen.

Üblicherweise setzt man ein cis- und trans-4-(2,2,3,3-Tetrafluorpropoxy)-zimtsäurenitril im Molverhältnis 75:25 bis 25:75, insbesondere 70:30 bis 50:50, bevorzugt 65:35 bis 60:40 enthaltendes Gemisch ein .

Man destilliert das cis-/trans-Isomerengemisch unter vergleichweise schonenden Bedingungen fraktioniert. Die zur Fraktionierung verwendete Destillationskolonne soll eine ausreichende Trennleistung, beispielsweise 15 bis 20 theoretische Böden aufweisen.

Es ist überraschend, daß trotz der vergleichweise hohen Temperaturen, die bei dieser Destillation aufgrund der starken Polarität und hohen Molmasse des cis- und trans-4-(2,2,3,3-Tetrafluorpropoxy)-zimtsäurenitrils unvermeidbar sind, keine Zersetzung oder eine allenfalls recht geringe Zersetzung auftritt. Es war ferner nicht vorauszusehen, daß es

gelingen würde, das gewünschte cis- oder trans-Isomere in einem derartigen Maße anzureichern, daß sich mittels einer weiteren Reinigungsstufe eine hohe bis sehr hohe Reinheit erzielen läßt. Eine Reinheit von wenigstens 95 % läßt sich ohne weiteres erreichen. Auch eine Reinheit von wenigstens 97,5 % ist nicht ungewöhnlich. Selbst Reinheiten, wie sie für die Weiterverarbeitung der Isomeren zu pharmazeutischen wirksamen Stoffen erforderlich sind, nämlich 99 % und mehr, lassen sich erreichen.

Bei der Destillation sind zu hohe Temperaturen, ebenso wie zu lange Verweilzeiten zu vermeiden, da zu hohe Temperaturen ebenso wie zu lange Verweilzeiten zur Bildung unerwünschter Nebenprodukte führen, die nur sehr schwer oder gar nicht aus dem gewünschten Wertprodukt entfernbar sind.

Man führt die Destillation unter reduziertem Druck, üblicherweise bei 0,01 bis 50, insbesondere 1 bis 30, bevorzugt 5 bis 10 mbar durch. Um eine überhöhte Belastung zu vermeiden, empfiehlt es sich, bei der Destillation kein allzu hohes Rücklaufverhältnis zu wählen. Im allgemeinen erweist sich ein Rücklaufverhältnis von 2:1 bis 1:4 insbesondere von 1:1 bis 1:2 als ausreichend.

Man wählt die Destillationsbedingungen so, daß man eine wenigstens 80 Gew.-% cis-4-(2,2,3,3-Tetrafluorpropoxy)-zimtsäurenitril und/oder eine wenigstens 80 Gew.-% trans-4-(2,2,3,3-Tetrafluorzimtsäurentril) enthaltende Hauptfraktion abtrennen kann. Im Verlauf der Destillation geht das cis-4-(2,2,3,3-Tetrafluorpropoxy)-zimtsäurenitril als erstes Produkt über, das trans-4-(2,2,3,3-Tetrafluorpropoxy)-zimtsäurenitril reichert sich im Sumpfprodukt an. Man kann das trans-4-(2,2,3,3-Tetrafluorpropoxy)-zimtsäurenitril aus dem Sumpfprodukt abdestillieren und das Destillat als Hauptfraktion oder auch ein entsprechend angereichertes Sumpfprodukt als Hauptfraktion weiterverarbeiten.

Man trennt üblicherweise eine 80 bis 95 Gew.-%, insbesondere 85 bis 90 Gew.-% cis-4-(2,2,3,3-Tetrafluorpropoxy)-zimtsäurenitril und eine 80 bis 95 Gew-%, insbesondere 85 bis 90 Gew.-% trans-4-(2,2,3,3-Tetrafluorpropoxy)-zimtsäurenitril enthaltende Hauptfraktion ab.

Neben den Hauptfraktion fallen weitere Fraktionen an. Diese übrigen Fraktionen sind Vorlauffraktionen, Zwischenlauffraktionen und Destillationsrückstände.

Zur weiteren Reinigung unterwirft man die Hauptfraktion entweder einer fraktionierten Kristallisation oder einer Schmelzkristallisation. Die Technik der Schmelzkristallisation ist beispielsweise in Ullmann's Encyclopedia of Industrial Chemistry, Vol. B2, Seiten 3-29 bis 3-34 5th Edition (1988) näher beschrieben.

Um fraktioniert zu kristallisieren, löst man das zu reinigende Produkt (Hauptfraktion) gegebenenfalls bei erhöhter Temperatur in einem organischen Lösungsmittel auf und führt die Kristallisation durch Einengen und/oder Kühlen herbei.

Die Temperatur, bei der man das zu reinigende Produkt auflöst, hängt auch vom jeweils verwendeten Lösungsmittel und der jeweiligen Konzentration der dabei entstehenden Lösung ab.

Im allgemeinen löst man das zu reinigende Produkt (Hauptfraktion) bei 20 bis 180, insbesondere 60 bis 150°C. Als Lösungsmittel eignen sich halogenierte oder nichthalogenierte aromatische oder aliphatische Kohlenwasserstoffe, Alkohole, Alkylether oder Alkylester. Auch ein Gemisch dieser Lösungsmittel läßt sich verwenden.

Ohne Anspruch auf Vollständigkeit zu erheben seien als geeignete Lösungsmittel Toluol, o-Xylol, m-Xylol, p-Xylol, Gemische isomerer Xylole, Chlorbenzol, Chlortoluol, Hexan, Heptan, Octan, Gemische verschiedener aliphatischer Kohlenwasserstoffe mit 5 bis 10 Kohlenstoffatomen, Dichlormethan, Trichlormethan, 1,2-Dichlorethan, Methanol, Ethanol, n-Propanol, i-Propanol, n-Butanol, i-Butanol, Gemische aus aliphatischen Alkoholen mit 1 bis 3 Kohlenstoffatomen mit Wasser, Methyl-tert.-butylether, Diethylether, Di-n-propylether, Di-n-butylether, Ethylacetat, Propylacetat, Butylacetat oder Mischungen der vorstehend genannten Lösungsmittel genannt.

Besonders geeignete Lösungsmittel sind Toluol, o-Xylol, m-Xylol, p-Xylol, Gemische isomerer Xylole, Chlorbenzol, Chlortoluol, Dichlormethan, Trichlormethan, 1,2-Dichlorethan, Methanol, Ethanol, n- und i-Propanol, n- und i-Butanol und Mischungen der vorstehend genannten Solventien.

Eine andere Möglichkeit, die Hauptfraktion weiter zu reinigen, eröffnet die sogenannte Schmelzkristallisation, deren unterschiedliche Varianten, wie bereits zuvor erwähnt, beispielsweise in Ullmann's Encyclopedia of Industrial Chemistry, Vol. B2, Seiten 3-29 bis 3-34, 5th Edition (1988) erläutert werden.

Um die Schmelzkristallisation durchzuführen, läßt man die Hauptfraktionen kristallisieren, setzt sie in kristallisiertem Zustand einer allmählichen Temperatursteigerung aus und trennt die dabei entstehenden schmelzflüssigen Bestandteile ab.

Einer speziellen Variante zufolge füllt man das zu reinigende Produkt (Hauptfraktion) in ein senkrecht stehendes, thermostatisierbares Rohr, beispielsweise in eine leere Destillationskolonne, ein, und bringt das Produkt, beispielsweise durch Kühlen und gegebenenfalls unter Zusatz von Impfkristallen zur Kristallisation.

Anschließend erwärmt man das senkrecht stehende Rohr mittels der Thermostatisierungsvorrichtung allmählich und steigert die Temperatur behutsam. Infolge der Wärmeeinwirkung entstehen schmelzflüssige Bestandteile, die aus der kristallisierten Hauptfraktion austreten und abtropfen. In den austretenden Tropfen ist bevorzugt das eine Isomere enthalten, während sich das andere Isomere bevorzugt in dem verbleibenden Rückstand anreichert. Auf diese Weise lassen sich die aus der Destillation anfallenden Hauptfraktionen, die entweder das cis-4-(2,2,3,3-Tetrafluorpropoxy)-zimtsäurenitril oder das trans-4-(2,2,3,3-Tetrafluorpropoxy)-zimtsäurenitril in angereicherter Form enthalten, auf schonende Weise weiter reinigen.

Besonders überraschend ist es, daß bei Reinigung einer trans-4-(2,2,3,3-Tetrafluorpropoxy)-zimtsäurenitril enthaltenden Hauptfraktion mittels Schmelzkristallisation nicht das bei höherer Temperatur schmelzende cis-4-(2,2,3,3-Tetrafluorpropoxy)-zimtsäurenitril, sondern das bei niedrigeren Temperaturen schmelzende trans-4-(2,2,3,3-Tetrafluorpropoxy)-zimtsäurenitril als Kristallisat im Rückstand verbleibt, während das bei höherer Temperatur schmelzende cis-4-(2,2,3,3-Tetrafluorpropoxy)-zimtsäurenitril in angereicherter Form schmelzflüssig abtropft.

Demzufolge eignet sich die Schmelzkristallisation in besonderem Maße zur Herstellung von reinem trans-4-(2,2,3,3-Tetrafluorpropoxy)-zimtsäurenitril.

Das erfindungsgemäße Verfahren gestattet, wie zuvor bereits erläutert, durch Umwandlung (Isomerisierung) des jeweils nicht benötigten Isomeren oder Isomerengemisches in das gewünschte Isomere oder Isomerengemisch wahlweise das cis-4-(2,2,3,3-Tetrafluorpropoxy)-zimtsäurenitril oder das trans-4-(2,2,3,3-Tetrafluorpropoxy)-zimtsäurenitril herzustellen . Man unterwirft, falls erforderlich, eine der beiden Hauptfraktionen, nämlich diejenige, die das nichterwünschte Isomere enthält, und/oder die übrigen Fraktionen, die je nach ihrer Herkunft als Vorlauffraktionen, Zwischenlauffraktionen oder Destillationsrückstand das cis- und trans-Isomere in unterschiedlichen Verhältnissen enthalten, mittels Licht einer photochemischen Isomerisierung und/oder mittels Wärme in Gegenwart einer Base einer thermischen Isomerisierung.

Bei der photochemischen Isomerisierung wird das zu isomerisierenden Material mit Hilfe einer geeigneten Lichtquelle, beispielsweise eines üblichen UV-Strahlers oder literaturbekannter Belichtungsapparaturen, die beispielsweise mit Quecksilber-Hochdruck- oder Quecksilber-Mitteldrucklampen bestückt sind, bestrahlt. Zur Technik der Bestrahlung sei auf J. Kagan, Organic Photochemistry, Principles and Applications (Academic Press, London (1993)) hingewiesen.

Üblicherweise genügt es, die Isomerisierung mit Licht einer Wellenlänge von 100 bis 500, insbesondere 150 bis 360, bevorzugt 180 bis 320 nm durchzuführen. Beabsichtigt man thermisch zu isomerisieren, so führt die Isomerisierung normalerweise bei 30 bis 170, insbesondere 60 bis 140°C, in Gegenwart einer stickstoffhaltigen Base oder eines Gemisches stickstoffhaltiger Basen durch. Man setzt als Base eine stickstoffhaltige, heterocyclische Verbindung, ein aliphatisches, cycloaliphatisches, araliphatisches oder aromatisches Amin oder ein Gemisch dieser Stoffe ein. Ohne Anspruch auf Vollständigkeit zu erheben, seien als geeignete Basen Pyrrolidon, Piperidin, Piperazin, Pyridin, Morpholin, Triethylamin, Tripropylamin, Tributylamin, ein Dialkylanilin, Anilin, Benzylamin oder ein Gemisch dieser Stoffe genannt.

Man kann die Isomerisierung auschließlich mittels Licht oder ausschließlich mittels Wärme und in Gegenwart einer Base durchführen. Es ist jedoch auch möglich, diese beiden Arten der Isomerisierung in beliebiger Reihenfolge nacheinander oder auch gleichzeitig anzuwenden.

Je nach Art und Länge der Isomerisierung stellt sich ein dem jeweiligen thermodynamischen Gleichgewicht entsprechendes Isomerenverhältnis ein. Das isomerisierte Produkt wird anschließend in das Verfahren wiedereingesetzt und der fraktionierten Destillation zugeführt.

Bei Durchführung des erfindungsgemäßen Verfahrens fallen auch Fraktionen an, die nicht isomerisiert werden müssen. Diese Produktgemische lassen sich direkt in das Verfahren wiedereinsetzen und können direkt der fraktionierten Destillation zugeleitet werden.

Das erfindungsgemäße Verfahren läßt sich kontinuierlich oder diskontinuierlich durchführen. Besonders einfach gestaltet sich eine diskontinuierliche Durchführung des Verfahren.

Die nachfolgenden Beispiele beschreiben die Erfindung, ohne sie zu beschränken.

Experimenteller Teil:

Herstellung von 4-(2,2,3,3-Tetrafluorpropoxy)-zimtsäurenitril (cis-/trans-Isomerengemisch)

Man erhitzt ein Gemisch aus 500 g 4-(2,2,3,3-Tetrafluorpropoxy)benzaldehyd, 183 g Cyanessigsäure, 250 ml Pyridin und 250 ml Piperidin unter Rühren und läßt 1 Stunde bei 110°C reagieren. Anschließend setzt man weitere 36,6 g Cyanessigsäure zu und läßt weitere 30 Minuten bei 110°C reagieren. Man kühlt die Reaktionsmischung ab, destilliert unter reduziertem Druck und erhält 433,1 g 4-(2,2,3,3-Tetrafluorpropoxy)zimtsäurenitril (Ausbeute: 78 %; Reinheit 95 %) in Form eines Isomerengemisches, das cis-4-(2,2,3,3-Tetrafluorpropoxy)-zimtsäurenitril und trans-4-(2,2,3,3-Tetrafluorpropoxy)-zimtsäurenitril im Molverhältnis cis:trans von etwa 1,35 bis 1,8:1) (entsprechend einem Gewichtsverhältnis von etwa (58 bis 64): (42 bis 36)) und daneben etwa bis 3 Gew.-% Piperidin und 2 Gew.-% nicht identifizierte Verunreinigungen und Harze enthält.

Beispiel 1

Fraktionierte Destillation von 4-(2,2,3,3-Tetrafluorpropoxy)-zimtsäurenitril (cis-/trans-Isomerengemisch)

Die fraktionierte Destillation wird mit einer verspiegelten Fraktionierkolonne von 100 cm Länge (Durchmesser 5 cm), die mit Füllkörpern (Fabrikat Sulzer CX) bestückt ist, durchgeführt. Die Trennleistung dieser Kolonnne entspricht - ermittelt durch Probedestillation eines Testgemisches - 15 bis 18 Böden.

Es werden 897 g eines gemäß Beispiel 1 hergestellten cis-/trans-Isomerengemisches, das cis-4-(2,2,3,3-Tetrafluorpropoxy)-zimtsäurenitril und trans-4-(2,2,3,3-Tetrafluorpropoxy)-zimtsäurenitril im Molverhältnis cis:trans von etwa (1,35 bis 1,8):1 (entsprechend einem Gewichtsverhältnis von etwa (58 bis 64):(42 bis 36)), und daneben etwa bis zu 3 Gew.-% Piperidin und 2 Gew.-% nicht identifizierte Verunreinigungen und Harze enthält, eingesetzt.
Die Steuerung des abgenommenen Produktstroms und des jeweils anfallenden Rücklaufs erfolgt über ein Flüssigkeitsteiler-Aggregat.

Die angegebenen Drücke und Temperaturen beziehen sich stets auf den Kopf der Fraktionierkolonne.

1. Fraktion: Bis 5 Torr (6,7 mbar) und 136 bis 138°C fallen 23,9 g, die 62,2 Gew.-% cis-4-(2,2,3,3-Tetrafluorpropoxy)-zimtsäurenitril enthalten, an. Das Rücklaufverhältnis beträgt 3:2.

2. Fraktion: Ab 5 Torr (6,7 mbar) und 138°C bis 5 Torr (6,7 mbar) und 158°C fallen 356,0 g einer Hauptfraktion, die 81,3 Gew.-% cis-4-(2,2,3,3-Tetrafluorpropoxy)-zimtsäurenitril und 18,7 Gew.-% trans-4-(2,2,3,3-Tetrafluorpropoxy)-zimtsäurenitril enthält, an. Das Rücklaufverhältnis beträgt 3:2.

3. Fraktion: Bei 4 Torr (5,3 mbar) und 158 bis 161°C fallen 111,0 g Zwischenlauf, die jeweils 50 Gew.-% cis- und 50 Gew.-trans-4-(2,2,3,3-Tetrafluorpropoxy)-zimtsäurenitril enthalten, an. Das Rücklaufverhältnis beträgt 1:3.

4. Fraktion: Bei 4 Torr (5,3 mbar) und 161°C fallen 49,2 g Zwischenläufe, die 76,2 Gew.-% trans-4-(2,2,3,3-Tetrafluorpropoxy)-zimtsäurenitril und 23,8 Gew.-% cis-4-(2,2,3,3-Tetrafluorpropoxy-zimtsäurenitril enthalten, an. Das Rücklaufverhältnis beträgt 1:1.

5. Fraktion: Ab 3 Torr (4 mbar) und 159°C bis 3 Torr (4 mbar) und 164°C fallen 209,6 g einer Hauptfraktion, die 80,5 Gew.-% trans-4-(2,2,3,3-Tetrafluorpropoxy)-zimtsäure und 19,5 Gew.-% cis-4-(2,2,3,3-Tetrafluorpropoxy)-zimtsäurenitril enthält, an. Das Rücklaufverhältnis beträgt 1:1.

6. Rückstand: Nach Abtrennung der Hauptfraktion wird die Destillation beendet, um die thermische Belastung des Sumpfproduktes nicht weiter zu erhöhen. Es verbleiben 117 g Sumpfprodukt, das etwa 80 Gew.-% 4-(2,2,3,3-Tetrafluorpropoxy)-zimtsäurenitril (Verhältnis cis-/trans-Isomer etwa 1:4) und etwa 20 Gew.- % nicht identifizierte Stoffe enthält. In den Füllkörpern der Kolonne befinden sich noch etwa 30 g Produkt.

Sowohl das Sumpfprodukt als auch das in den Füllkörpern der Kolonne noch vorhandene Produkt lassen sich in einer weiteren fraktionierten Destillation einsetzen.

Der Zersetzungsgrad beträgt etwa 3 % des Einsatzproduktes (ermittelt durch kalibrierte gaschromatographische Analyse) und liegt damit recht niedrig. Im Sumpf sind noch keine Verkokungen an der Wand, wie sie bei fraktionierter Destillation vergleichbarer Produkte auftreten, zu beobachten, was als Indiz für eine hinreichend schonende Durchführung der fraktionierten Destillation zu bewerten ist.

Beispiel 3a

Fraktionierte Kristallisation einer trans-4-(2,2,3,3-Tetrafluorpropoxy)-zimtsäurenitril enthaltenden Hauptfraktion

100 g der aus Beispiel 2 als 5. Fraktion anfallenden Hauptfraktion werden bei 100 bis 140°C in 300 g Xylol gelöst. Anschließend läßt man langsam bis auf 20°C abkühlen und trennt das Kristallisat von der Mutterlauge. Man wiederholt diese Operation zwei- bis dreimal, wobei man zunächst 280, dann 230 und schließlich 200 g Xylol verwendet. Auf diese Weise läßt sich trans-4-(2,2,3,3-Tetrafluorpropoxy)-zimtsäurenitril in einer Reinheit von 98 % und in 75 % Ausbeute (≙ 61,5 g) gewinnen.

Beispiel 3b

Fraktionierte Kristallisation einer cis-4-(2,2,3,3-Tetrafluorpropoxy)-zimtsäurenitril enthaltenden Hauptfraktion

Man arbeitet wie in Beispiel 3 angegeben, setzt jedoch 100 g einer analog zu Beispiel 2 hergestellten Hauptfraktion, die 80 Gew.-% cis-4-(2,2,3,3-Tetrafluorpropoxy)-zimtsäurenitril enthält, ein. Durch mehrfache Wiederholung der Kristallisation erhält man cis-4-(2,2,3,3-Tetrafluorpropoxy)-zimtsäurenitril in einer Reinheit von 98,4 % und in einer Ausbeute von 76,3 % (60,1 g).

Beispiel 4a

Schmelzkristallisation einer trans-4-(2,2,3,3-Tetrafluorpropoxy)-zimtsäurenitril enthaltenden Hauptfraktion

Man setzt 200 g einer analog zu Beispiel 2 hergestellten Hauptfraktion, die 80 Gew.-% trans-4-(2,2,3,3-Tetrafluorpropoxy)-zimtsäurenitril enthält, in eine senkrecht stehende, thermostatisierbare Kolonne (Durchmesser: 4 cm) ein und kristallisiert unter Abkühlen auf 5°C. Um die Kristallisation in der Kolonne zu unterstützen, kann man Impfkristalle zusetzen. Die Kolonne enthält bis auf ein Ablaßventil am Boden keine Einbauten.
Nach erfolgter Kristallisation beginnt man mit Hilfe der Thermostatisierungsvorrichtung zu erwärmen. Die Schmelzkristallisation setzt bei einer Temperatur von 42 bis 45°C ein und wird bis zu einer Temperatur von 50 bis 52°C durchgeführt. Die mittels Schmelzkristallisation einer trans-4-(2,2,3,3-Tetrafluorpropoxy)-zimtsäurenitril-Hauptfraktion erzielbare Ausbeute an reinem trans-4-(2,2,3,3-Tetrafluorpropoxy)-zimtsäurenitril liegt üblicherweise niedriger als die mittels Schmelzkristallisation einer cis-4-(2,2,3,3-Tetrafluorpropoxy)-zimtsäurenitril-Hauptfraktion erreichbare Ausbeute an cis-Verbindung, da die trans-Verbindung (Erstarrungspunkt: 50,6°C, gemessen an < 99 %ig reinem trans-4-(2,2,3,3-Tetrafluorpropoxy)-zimtsäurenitril) gegenüber der cis-Verbindung (Erstarrungspunkt: 53,95°C, gemessen an < 99 %ig reinem cis-4-(2,2,3,3-Tetrafluorpropoxy)-zimtsäurenitril) einen niedrigeren Schmelzpunkt bzw. Erstarrungspunkt besitzt. Überraschenderweise beginnt jedoch bei Einsatz einer trans-4-(2,2,3,3-Tetrafluorpropoxy)-zimtsäurenitril Hauptfraktion ab einer Temperatur von etwa 42 bis 44°C zunächst das bei höherer Temperatur schmelzende cis-4-(2,2,3,3-Tetrafluorpropoxy)-zimtsäurenitril abzutropfen, wobei sich im Rückstand das trans-Isomere anreichert.

Man erhält etwa 50 % des Einsatzes in Form verschiedener Fraktionen, die zwischen etwa 40 bis 90 Gew-% trans-4-(2,2,3,3-Tetrafluorpropoxy)-zimtsäurenitril und entsprechend 60 bis 10 Gew-% cis-4-(2,2,3,3-Tetrafluorpropoxy)-zimtsäurenitril enthalten.

Das verbleibende Material enthält weniger als 5 Gew.-% des cis-Isomeren und mehr als 95 Gew.-% trans-4-(2,2,3,3-Tetrafluorpropoxy)-zimtsäurenitril

Beispiel 4b

Schmelzkristallisation einer cis-4-(2,2,3,3-Tetrafluorpropoxy)-zimtsäurenitril enthaltenden Hauptfraktion

Man setzt 200 g einer analog zu Beispiel 2 hergestellten Hauptfraktion, die 80 Gew.-% cis-4-(2,2,3,3-Tetrafluorpropoxy)-zimtsäurenitril enthält in eine senkrecht stehende, thermostatisierbare Kolonne (Durchmesser 4 cm) ein und kristallisiert unter Abkühlen auf 5°C. Um die Kristallisation in der Kolonne zu unterstützen, kann man Impfkristalle zusetzen. Die Kolonne enthält bis auf ein Ablaßventil am Boden keine Einbauten.
Nach erfolgter Kristallisation beginnt man mit Hilfe der Thermostatisierungsvorrichtung zu erwärmen. Die Schmelzkristallisation setzt bei einer Temperatur von 42 bis 45°C ein und wird bis zu einer Temperatur von 52 bis 54°C durchgeführt. Die Ausbeute an reinem cis-4-(2,2,3,3-Tetrafluorpropoxy)-zimtsäurenitril liegt üblicherweise höher als die Ausbeute an trans-Verbindung, da die cis-Verbindung (Erstarrungspunkt: 53,95°C, gemessen an < 99 %ig reinem cis-4-(2,2,3,3-Tetrafluorpropoxy)-zimtsäurenitril) gegenüber der trans-Verbindung (Erstarrungspunkt: 50,6 °C, gemessen an < 99 %ig reinem trans-4-(2,2,3,3-Tetrafluorpropoxy)-zimtsäurenitril) einen höheren Schmelzpunkt bzw. Erstarrungspunkt besitzt. Erwartungsgemäß beginnt bei Steigerung der Temperatur ab etwa 42 bis 44°C zunächst das bei niedriger Temperatur schmelzende trans-4-(2,2,3,3-Tetrafluorpropoxy)-zimtsäurenitril abzutropfen, wobei sich im Rückstand das cis-Isomere anreichert.

Man erhält etwa 40 % des Einsatzes in Form verschiedener Fraktionen, die zwischen etwa 40 bis 90 Gew.-% cis-4-(2,2,3,3-Tetrafluorpropoxy)-zimtsäurenitril und entsprechend 60 bis 10 Gew.-% trans-4-(2,2,3,3-Tetrafluorpropoxy)-zimtsäurenitril enthalten.
Das verbleibende Material (118 g) enthält weniger als 5 Gew.-% trans-4-(2,2,3,3-Tetrafluorpropoxy)-zimtsäurenitril und mehr als 95 Gew.-% cis-4-(2,2,3,3-Tetrafluorpropoxy)-zimtsäurenitril.
Setzt man die Schmelzkristallisation fort und nimmt man eine geringere Ausbeute (60 g ≙ 30 %) in Kauf, so läßt sich die Reinheit des cis-4-(2,2,3,3-Tetrafluorpropoxy)-zimtsäurenitril auf 99 % und darüber steigern.

Nachfolgend sind die cis-/trans-Isomeren anhand spektroskopischer Daten näher charakterisiert, wobei sich alle Angaben jeweils auf ein Produkt mit > 99 %iger Reinheit beziehen.

cis-4-(2,2,3,3-Tetrafluorpropoxy)-zimtsäurenitril (Reinheit > 99 Gew.-%):
Erstarrungspunkt: 53,95°C

| | |
|---|---|
| $^1$H-NMR ($CDCl_3$, TMS): | 4,40 (ttr, J = 11,5, 1,5 Hz, $OC\underline{H}_2$-$CF_2$) |
| | 5,37 (d, J = 12,1 Hz, = C$\underline{H}$-CN) |
| | 6,06 (dt, J = 52,4, 4,8 Hz, -$CF_2\underline{H}$) |
| | 6,98 (m, J = 8,8 Hz, Ar-$\underline{H}^{3,5}$) |
| | 7,06 (d, J = 12,1 Hz Ar-C$\underline{H}$=) |
| | 7,82 (m, J = 8,8 Hz, Ar-$\underline{H}^{2,6}$) |
| $^{19}$F-NMR ($CDCl_3$, $CFCl_3$): | -125,3 (m, J = 11,5, 4,8 Hz, -C$\underline{F}_2$-) |
| | -139,6 (dtr, J = 52,4, 1,5 Hz, -C$\underline{F}_2$H) |

GC-MS (Reingehalt > 99,5 %), m/z (%): 45 (2,7), 46 (2,3), 50 (14,0), 51 (85,6), 52 (5,8), 53 (2,5), 61 (4,9), 62 (15,4), 63 (42,7), 64 (23,3), 65 (9,6), 66 (2,9), 69 (3,4), 74 (9,4), 75 (20,7), 76 (11,1), 77 (21,6), 78 (3,0), 82 (2,8), 86 (1,1), 87 (4,4), 88 (12,7), 89 (100), 90 (22,7), 91 (2,4), 92 (1,8), 95 (3,8), 99 (2,1), 100 (2,4), 101 (46,2), 102 (14,2), 103 (23,6), 104 (3,0), 114 (6,1), 115 (9,4), 116 (81,3), 117 (13,1), 118 (2,7), 126 (1,4), 127 (8,7), 128 (60,8), 129 (6,8), 130 (26,8), 131 (7,1), 140 (1,1), 144 (29,5), 145 (6,4), 158 (56,8), 159 (6,7), 160 (2,3), 200 (5,2), 259 (96,9, M*), 260 (13,6)
trans-4-(2,2,3,3-Tetrafluorpropoxy)-zimtsäurenitril (Reinheit > 99 Gew.-%):
Erstarrungspunkt: 50,6°C

| | |
|---|---|
| $^1$H-NMR ($CDCl_3$, TMS): | 4,39 (ttr, J = 11,5, 1,3 Hz, $OC\underline{H}_2$-$CF_2$) |
| | 5,77 (d, J = 16,7 Hz, = C$\underline{H}$-CN) |
| | 6,05 (dt, J = 52,6, 4,6 Hz, -$CF_2\underline{H}$) |
| | 6,96 (m, J = 8,7 Hz, Ar-$\underline{H}^{3,5}$) |
| | 7,34 (d, J = 16,7 Hz, Ar-C$\underline{H}$=) |
| | 7,44 (m, J = 8,7 Hz, Ar-$\underline{H}^{2,6}$) |
| $^{19}$F-NMR ($CDCl_3$, $CFCl_3$): | -125,1 (m, J = 11,5, 4,6, -C$\underline{F}_2$-) |
| | -139,4 (dtr, J = 52,6, 1,3, C$\underline{F}_2$H) |

GC-MS (Reingehalt > 99,5 %), m/z (%): 45 (3,7), 46 (2,8) 50 (14,6), 51 (85,7), 52 (5,6), 53 (3,0), 61 (5,6), 62 (17,3), 63 (42,9), 64 (25,4), 65 (11,6), 66 (1,4), 69 (3,6), 74 (9,7), 75 (20,1), 76 (14,0), 77 (24,0), 78 (3,6), 82 (3,1), 86 (2,2), 87 (7,7), 88 (12,1), 89 (100), 90 (24,8), 91 (2,6), 95 (3,4), 98 (1,1), 99 (2,7), 100 (7,3), 101 (49,0), 102 (15,2), 103 (24,6), 104 (2,9), 114 (6,1), 115 (8,0), 116 (84,8), 117 (14,1), 118 (3,5), 127 (9,1), 128 (57,6), 129 (7,4), 130 (29,9), 131 (7,3), 143 (1,0), 144 (33,2), 145 (6,5), 158 (56,8), 159 (6,6), 160 (2,0), 200 (4,3), 259 (93,1), 260 (12,3).

Beispiel 5a

Photochemische Isomerisierung einer trans-4-(2,2,3,3-Tetrafluorpropoxy)-zimtsäurenitril enthaltenden Hauptfraktion

5 g eines Isomerengemisches (82,63 Gew.-% trans- und 17,37 Gew.-% cis-4-(2,2,3,3-Tetrafluorpropoxy)-zimtsäurenitril) werden in 50 g Methanol gelöst und mit einem einfachen UV-Strahler belichtet.
Die nachfolgende Tabelle gibt den Verlauf der photochemischen Isomerisierung wieder. Es findet eine Umwandlung statt, die zu einem Gleichgewicht von etwa 60 Gew.-% cis- und etwa 40 Gew-% trans-4-(2,2,3,3-Tetrafluorpropoxy)-zimtsäurenitril führt.

Tabelle

| Belichtungsdauer (Stunden) | cis-Isomeres[1] in Gew.-% | trans-Isomeres[2] in Gew.-% |
|---|---|---|
| 0 | 17,37 | 82,63 |
| 1 | 21,5 | 78,5 |
| 7 | 35,6 | 64,4 |
| 11 | 43,4 | 56,6 |
| 17 | 47,8 | 52,2 |
| 25 | 53,2 | 46,8 |
| 33 | 58,07 | 41,93 |
| 41 | 59,15 | 40,85 |
| 57 | 59,4 | 40,6 |

[1] cis-Isomeres = cis-4-(2,2,3,3-Tetrafluorpropoxy)-zimtsäurenitril
[2] trans-Isomeres = trans-4-(2,2,3,3-Tetrafluorpropoxy)-zimtsäurenitril

Wie aus der Tabelle hervorgeht, ist nach etwa 30 Stunden Belichtungsdauer der Gleichgewichtszustand nahezu erreicht.
Während der Umsetzung findet eine Zersetzung nur in untergeordnetem Maße statt. Die methanolische Lösung verfärbt sich von hellgelb nach orangefarben. Analoge photochemische Isomerisierungen von Gemischen beliebiger cis-/trans-Isomerenzusammensetzung führen stets zu einem Produkt, das den zuvor erwähnten Gleichgewichtszustand von etwa 60 Gew.-% cis- und etwa 40 Gew.-% trans-4-(2,2,3,3-Tetrafluorpropoxy)-zimtsäurenitril aufweist.

Beispiel 5b

Thermische Isomerisierung einer cis-4-(2,2,3,3-Tetrafluorpropoxy)-zimtsäurenitril enthaltenden Hauptfraktion

100 g einer cis-4-(2,2,3,3-Tetrafluorpropoxyl)-zimtsäurenitril enthaltenden Hauptfraktion (81,3 Gew.-% cis- und 18,7 Gew.-% trans-4-(2,2,3,3-Tetrafluorpropoxy)-zimtsäurenitril) werden mit 5 g Pyridin und 5 g Piperidin versetzt und zum Rückfluß erhitzt.

Nach 5 Stunden erhält man eine Mischung die das cis- und trans-Isomere im Verhältnis 1,8:1 (entsprechend 64,3 Gew.-% cis-4-(2,2,3,3-Tetrafluorpropoxy)-zimtsäurenitril und 35,7 Gew.-% trans-4-(2,2,3,3-Tetrafluorpropoxy)-zimtsäurenitril) aufweist.
Diese thermisch isomerisierte Mischung läßt sich mittels fraktionierter Destillation wie in Beispiel 1 angegeben, erneut auftrennen und die dabei anfallenden Hauptfraktionen lassen sich mittels fraktionierter Kristallisation oder Schmelzkristallisation weiter reinigen.
Durch mehrmalige Wiederholung des erfindungsgemäßen Verfahrens ist es möglich, das gesamte cis-4-(2,2,3,3-Tetrafluorpropoxy)-zimtsäurenitril in reines trans-4-(2,2,3,3-Tetrafluorpropoxy)-zimtsäurenitril zu überführen.

Beispiel 6

Herstellung von 4-(2,2,3,3-Tetrafluorpropoxy)-zimtsäurenitril unter Zusatz einer cis-4-(2,2,3,3-Tetrafluorpropoxy)-zimtsäurenitril enthaltenden Hauptfraktion und thermische Isomerisierung

735,0 g (3, 12 mol) 4-(2,2,3,3-Tetrafluorpropoxy)-zimtsäurenitril-benzaldehyd, 269 g Cyanessigsäure, 660 g cis-4-(2,2,3,3-Tetrafluorpropoxy)-zimtsäurenitril (Hauptfraktionen mit 80 bis 85 Gew.-% cis-Isomeren) werden mit 750 g Xylol, 750 ml Pyridin und 750 ml Piperidin versetzt und unter Rühren auf 110°C erhitzt. Man läßt 2 Stunden reagieren, setzt anschließend erneut 53,8 g Cyanessigsäure zu und läßt weitere 30 Minuten reagieren. Nach Abkühlen destilliert man das Reaktionsgemisch unter reduziertem Druck und erhält 1173 g 4-(2,2,3,3-Tetrafluorpropoxy)-zimtsäure (Ausbeute: 87 %; Reinheit > 95 Gew.-%) in Form eines Isomerengemisches, das cis-4-(2,2,3,3-Tetrafluorpropoxy)-zimtsäurenitril und trans-4-(2,2,3,3-Tetrafluorpropoxy)-zimtsäurenitril im Molverhältnis von etwa 1,8:1 und daneben etwa bis 3 Gew.-% Piperidin und bis 2 Gew.-% nicht identifizierbare Verunreinigungen enthält.

Wie ein Vergleich mit den Ergebnissen der photochemischen und thermischen Isomerisierungen (Beispiele 5a und 5b) zeigt, entspricht die erhaltene Reaktionsmischung nahezu dem für eine Isomerisierung typischen cis-/trans-Isomerenverhältnis. Die Reaktionsmischung weist ein cis-/trans-Isomerenverhältnis von 64,3 Gew-% : 35,7 Gew.-%, das gemäß den Beispielen 5a und 5b isomerisierte Produkt ein Isomerenverhältnis von etwa 60 Gew.-% cis-Isomeres zu etwa 40 Gew.-% trans-Isomeres auf.

Man destilliert die vorstehend beschriebene Reaktionsmischung analog Beispiel 1 und erhält 489 g einer Hauptfraktion, die 82 Gew.-% trans-4-(2,2,3,3-Tetrafluorpropoxy)-zimtsäurenitril und 18 Gew.-% cis-4-(2,2,3,3-Tetrafluorpropoxy)-zimtsäurenitril enthält.

Die gesamten Vorlauffraktionen und das Sumpfprodukt (etwa 650 bis 675 g) werden einer Isomerisierung unterworfen und in die fraktionierte Destillation wiedereingesetzt. Es ist auch möglich, diese Produkte in die eigentliche Synthese einzusetzen und im Verlauf der Synthese zu isomerisieren.

Beispiel 7

Schmelzkristallisation der aus Beispiel 6 erhaltenen trans-4-(2,2,3,3-Tetrafluorpropoxy)-zimtsäurenitril-Hauptfraktion

Man füllt die gemäß Beispiel 6 erhaltenen 489 g Hauptfraktion (82 Gew.-% trans-4-(2,2,3,3-Tetrafluorpropoxy)-zimtsäurenitril:
18 Gew.-% cis-4-(2,2,3,3-Tetrafluorpropoxy)-zimtsäurenitril) wie in Beispiel 4a beschrieben in eine senkrecht stehende, thermostatisierbare Kolonne ohne Einbauten. Nach Kristallisation bei 5°C erhöht man die Temperatur allmählich und nimmt folgende Fraktionen ab:

| | | |
|---|---|---|
| bis 45°C | 72 g | (38 Gew.-% trans-Isomeres) |
| 45 bis 47°C | 33 g | (54 Gew.-% trans-Isomeres) |
| 47 bis 48°C | 22 g | (72 Gew.-% trans-Isomeres) |
| 48 bis 49°C | 15 g | (81 Gew.-% trans-Isomeres) |
| 49 bis 50°C | 12 g | (89 Gew.-% trans-Isomeres) |
| > 50°C | 335 g | (> 95 Gew.-% trans-Isomeres) |

**Patentansprüche**

1. Die Verbindungen cis-4-(2,2,3,3-Tetrafluorpropoxy)-zimtsäurenitril und trans-4-(2,2,3,3-Tetrafluorpropoxy)-zimtsäurenitril

2. cis-4-(2,2,3,3-Tetrafluorpropoxy)-zimtsäurenitril

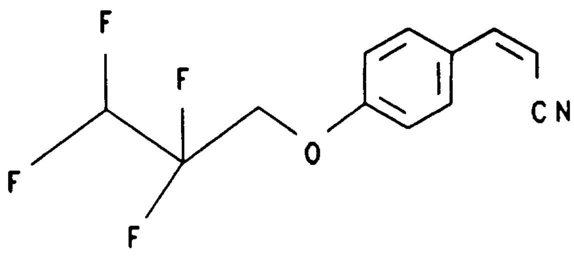

3. trans-4-(2,2,3,3-Tetrafluorpropoxy)-zimtsäurenitril

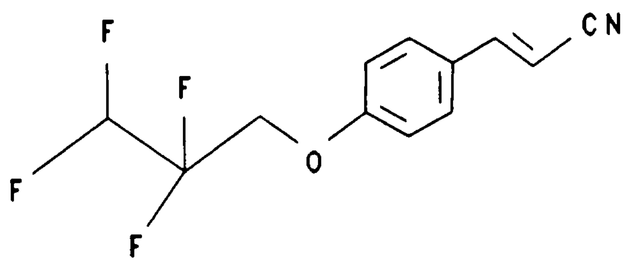

4. Verfahren zur Herstellung von cis-4-(2,2,3,3-Tetrafluorpropoxy)-zimtsäurenitril und trans-4-(2,2,3,3-Tetrafluorpropoxy)-zimtsäurenitril oder wahlweise einer dieser beiden Verbindungen, dadurch gekennzeichnet, daß man ein cis- und trans-4-(2,2,3,3-Tetrafluorpropoxy)-zimtsäurenitril enthaltendes Gemisch unter reduziertem Druck fraktioniert destilliert, eine mindestens 80 Gew.-% cis-4-(2,2,3,3-Tetrafluorpropoxy)-zimtsäurenitril und eine mindestens 80 Gew.-% trans-4-(2,2,3,3-Tetrafluorpropoxy)-zimtsäurenitril enthaltende Hauptfraktion abtrennt, diese Hauptfraktionen mittels fraktionierter Kristallisation oder Schmelzkristallisation weiter reinigt oder eine dieser Hauptfraktionen durch Isomerisierung auf das dem jeweiligen thermodynamischen Gleichgewicht entsprechende cis-/trans-Isomerenverhältnis einstellt und in das Verfahren zurückführt und die übrigen Fraktionen direkt oder nach Isomerisierung in das Verfahren zurückführt.

5. Verfahren nach Anspruch 4, dadurch gekennzeichnet, daß man ein cis- und trans-4-(2,2,3,3-Tetrafluorpropoxy)-zimtsäurenitril im Molverhältnis 75:25 bis 25:75, insbesondere 70:30 bis 50:50, bevorzugt 65:35 bis 60:40 enthaltendes Gemisch einsetzt.

6. Verfahren nach Anspruch 4 oder 5, dadurch gekennzeichnet, daß man das Gemisch bei 0,01 bis 50, insbesondere 1 bis 30, bevorzugt 5 bis 10 mbar fraktioniert destilliert.

7. Verfahren nach einem oder mehreren der Ansprüche 4 bis 6, dadurch gekennzeichnet, daß man eine 80 bis 95 % cis-4-(2,2,3,3-Tetrafluorpropoxy)-zimtsäurenitril und eine 80 bis 95 Gew.-% trans-4-(2,2,3,3-Tetrafluorpropoxy)-zimtsäurenitril enthaltende Hauptfraktion abtrennt.

8. Verfahren nach einem oder mehreren der Ansprüche 4 bis 7, dadurch gekennzeichnet, daß man eine 85 bis 90 Gew.-% cis-4-(2,2,3,3-Tetrafluorpropoxy)-zimtsäurenitril und eine 85 bis 90 Gew.-% trans-4-(2,2,3,3-Tetrafluorpropoxy)-zimtsäurenitril enthaltende Hauptfraktion abtrennt.

9. Verfahren nach einem oder mehreren der Ansprüche 4 bis 8, dadurch gekennzeichnet, daß man die Hauptfraktion bei 20 bis 180, insbesondere 60 bis 150 °C in einem organischen Lösungsmittel löst.

10. Verfahren nach einem oder mehreren der Ansprüche 4 bis 9, dadurch gekennzeichnet, daß man die Hauptfraktion in einem halogenierten oder nichthalogenierten aliphatischen oder aromatischen Kohlenwasserstoff, einem Alkohol, Alkylether, Alkylester oder einem Gemisch dieser Lösungsmittel löst und gegebenenfalls unter Kühlen fraktioniert kristalliert.

11. Verfahren nach einem oder mehreren der Ansprüche 4 bis 8, dadurch gekennzeichnet, daß man die Hauptfraktionen kristallisieren läßt, sie in kristallisiertem Zustand einer allmählichen Temperaturerhöhung aussetzt und die dabei entstehenden schmelzflüssigen Bestandteile abtrennt.

12. Verfahren nach einem oder mehreren der Ansprüche 4 bis 8, dadurch gekennzeichnet, daß man, falls erforderlich, eine der beiden Hauptfraktionen und die übrigen Fraktionen mittels Licht einer photochemischen Isomerisierung und/oder mittels Wärme in Gegenwart einer Base einer thermischen Isomerisierung unterwirft und in das Verfahren zurückführt.

13. Verfahren nach einem oder mehreren der Ansprüche 4 bis 8 und 12, dadurch gekennzeichnet, daß man die Isomerisierung mit Licht einer Wellenlänge von 100 bis 500 nm durchführt.

**14.** Verfahren nach einem oder mehreren der Ansprüche 4 bis 8 und 12 und 13, dadurch gekennzeichnet, daß man die Isomerisierung bei 30 bis 170, insbesondere 60 bis 140°C, in Gegenwart einer stickstoffhaltigen Base oder eines Gemisches stickstoffhaltiger Basen durchführt.

**15.** Verfahren nach einem oder mehreren der Ansprüche 4 bis 8 und 12 bis 14, dadurch gekennzeichnet, daß man als Base eine stickstoffhaltige heterocyclische Verbindung, ein aliphatisches, cycloaliphatisches, araliphatisches oder aromatisches Amin oder ein Gemisch dieser Stoffe einsetzt.

**16.** Verfahren nach einem oder mehreren der Ansprüche 4 bis 8 und 12 bis 15, dadurch gekennzeichnet, daß man Pyrrolidon, Piperidin, Piperazin, Pyridin, Morpholin, Triethylamin, Tripropylamin, Tributylamin, ein Dialkylanilin, Anilin, Benzylamin oder ein Gemisch dieser Stoffe als Base einsetzt.